# EUROPEAN PATENT SPECIFICATION

(11) **EP 4 085 832 B1**
(45) Date of publication and mention of the grant of the patent: **22.11.2023**
(21) Application number: 21175372.8
(22) Date of filing: 21.05.2021
(51) Int. Cl.: A61B 5/08, A61B 5/145, G01N 33/497

(54) **NON-INVASIVE DETERMINATION OF BLOOD GLUCOSE LEVELS**
NICHTINVASIVE BESTIMMUNG DES BLUTZUCKERSPIEGELS
DÉTERMINATION NON INVASIVE DES NIVEAUX DE GLUCOSE SANGUIN

(30) Priority: 04.05.2021 US 202163183674 P
(43) Date of publication of application: 09.11.2022
(73) Proprietor: Roche Diabetes Care GmbH, 68305 Mannheim (DE); F. Hoffmann-La Roche AG, 4070 Basel (CH)
(72) Inventor: FINK, Herbert, 68305 Mannheim (DE); MAIHOEFER, Tim, 68305 Mannheim (DE); SCHULAT, Jochen, 68305 Mannheim (DE); BENDER, Jeffrey M., Indianapolis, 46250 Indiana (US)
(74) Representative: Meinel, Anne Julia

(56) References cited:
- WO-A1-2019/058021
- US-A1- 2003 008 407
- RYDOSZ ARTUR: "A Negative Correlation Between Blood Glucose and Acetone Measured in Healthy and Type 1 Diabetes Mellitus Patient Breath", JOURNAL OF DIABETES SCIENCE AND TECHNOLOGY, vol. 9, no. 4, 17 February 2015 (2015-02-17), pages 881-884, XP055856949, US ISSN: 1932-2968, DOI: 10.1177/1932296815572366
- VAN DEN VELDE S ET AL: "GC-MS analysis of breath odor compounds in liver patients", JOURNAL OF CHROMATOGRAPHY B, ELSEVIER, AMSTERDAM, NL, vol. 875, no. 2, 15 November 2008 (2008-11-15), pages 344-348, XP025879919, ISSN: 1570-0232, DOI: 10.1016/J.JCHROMB.2008.08.031 [retrieved on 2008-09-17]
- DRABINSKA NATALIA ET AL: "A literature survey of all volatiles from healthy human breath and bodily fluids: the human volatilome", JOURNAL OF BREATH RESEARCH, [Online] vol. 15, no. 3, 21 April 2021 (2021-04-21) , page 034001, XP055856939, US ISSN: 1752-7155, DOI: 10.1088/1752-7163/abf1d0 Retrieved from the Internet: URL:https://iopscience.iop.org/article/10. 1088/1752-7163/abf1d0/pdf> -& Drabinska Natalia ET AL: "A literature survey of all volatiles from healthy human breath and bodily fluids: the human volatilome (Supplementary Information - Part 2)", Journal of Breath Research (15), 21 April 2021 (2021-04-21), pages 1-9, XP055856940, Retrieved from the Internet: URL:https://cfn-live-content-bucket-iop-or g.s3.amazonaws.com/journals/1752-7163/15/3 /034001/4/jbrabf1d0supp1.zip [retrieved on 2021-11-02]

## Description

### TECHNICAL FIELD

This disclosure relates generally to a non-invasive determination of a subject's blood glucose level based on detecting volatile organic markers in exhaled breath or other gaseous emanation from the subject.

### BACKGROUND/RELATED ART

The monitoring of blood glucose levels is crucial for diabetes management. A typical glucose measurement method involves piercing the skin, typically a finger, to draw blood and applying the blood to a chemically active disposable medium. To avoid the necessity of repeatedly piercing the skin different non-invasive blood glucose monitoring technologies have also been proposed.

An example of a non-invasive blood glucose monitoring method is disclosed in WO 2020/02989 A1, the method combining infrared measurement with simultaneous pressure reading. Still, with infrared spectroscopy it is difficult to meat an in-vivo accuracy required for a reliable blood glucose management.

Another example of a non-invasive blood glucose monitoring technology is described in US 7,076,371 B2. A volatile marker, which is characteristic of the disease, is detected in exhaled breath or other gaseous emanation from the person and the detected marker data is analyzed in an artificial neural network with a fuzzy filter. For determining blood glucose levels the markers shall be selected to measure the destruction or deterioration of cell membranes by lipid peroxidation or protein oxidation, e.g. propanol or acetone, as those substances from metabolic processes of intestinal bacteria are positively correlated to blood glucose levels.

In Trefz, P., Obermeier, J., Lehbrink, R. et al. "Exhaled volatile substances in children suffering from type 1 diabetes mellitus: results from a cross-sectional study." Scientific Report 9, 15707 (2019), https://doi.org/10.1038/s41598-019-52165-x, the measurements for the determination of blood glucose levels are focused on the exhalation of ethanol, acetone, isopropanol, dimethyl sulfide, isoprene, pentanal, and limonene as these compounds have been previously associated to the disturbed glucose homeostasis or reflect a metabolic link to diabetes type 1 related comorbidities, i.e. dyslipidemia and oxidative stress.

In Rydosz, Artur: "A Negative Correlation Between Blood Glucose and Acetone Measured in Healthy and Type 1 Diabetes Mellitus Patient Breath", JOURNAL OF DIABETES SCIENCE AND TECHNOLOGY, vol. 9, no. 4, 17 February 2015 (2015-02-17), pages 881-884, XP055856949, US ISSN: 1932-2968, DOl: 10.1177/1932296815572366 a negative correlation between an acetone concentration in exhaled breath and a blood glucose level is reported.

In Van den Velde, S. et al.: "GC-MS analysis of breath odor compounds in liver patients", JOURNAL OF CHROMATOGRAPHY B, ELSEVIER, AMSTERDAM, NL, vol. 875, no. 2, 15 November 2008 (2008-11-15), pages 344-348, XP025879919, ISSN: 1570-0232, DOl: 10. 1016/J .JCHROMB.2008.08.031 a decreased concentration of Indole in a subject's exhaled breath was identified as sign for a liver disease.

An example of a device for non-invasive blood glucose monitoring is a skin surface sampling system as described in U.S. Patent 10,143,447 B2 entitled "Skin surface sampling system". This system utilizes an elongated collection tube with a sampling head positioned on one end in contact with the patient's skin. A liquid supply absorbs volatile organic compounds (VOC) and semi-volatile organic compounds (SVOC) from the surface of the skin and collects the mixed liquid in a sample collection device. The system is operated by positioning the sampling head on the skin surface and then flushing the liquid supply through a set of channel grooves in the sampling head that direct the mixed liquid to the collection tube. Although providing a potentially non-invasive form of blood glucose level monitoring, the system requires a liquid capture system.

Despite of the advantages and the progress achieved by the above-mentioned developments, some significant technical challenges remain regarding responsiveness and reliability. It is therefore an objective of the present invention to provide a fast responding and robust blood glucose monitoring method.

### SUMMARY OF THE INVENTION

This problem is solved by a method for non-invasively determining a subject's blood glucose level with the features of claim 1 and a non-invasive blood glucose level monitoring system configured to execute said method. Preferred embodiments, which might be realized in an isolated fashion or in any arbitrary combination, are listed in the dependent claims.

As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

In a first aspect of the present invention, a method to determine a subject's blood glucose level non-invasively by analyzing an exhaled breath or another gaseous emanation from the subject is disclosed. The method comprises non-invasively detecting an amount of at least one volatile organic marker in the subject's exhaled breath or the subject's other gaseous emanation as marker data and determining the subject's blood glucose level based on the marker data. The volatile organic marker is selected from a group of markers for which the amount of the volatile organic marker is negatively correlated to the blood glucose level.

A volatile organic marker, also called volatile organic compound (VOC), is an organic chemical that has a high vapor pressure or rather a low boiling point. Thus, VOCs are volatile at rather low temperatures such as room temperature for example. The human body is a major source of VOCs that originate from different parts and processes within the body. So-called endogenous VOCs originate from metabolic processes within the body. An important organ involved in generating and transforming such endogenous VOCs is the liver. VOCs originating from the environment are called exogenous VOCs, e.g. entering the body via respiratory air, food intake or diffusion through skin or originating from the microbiome, e.g. of the digestive tract.

VOCs generated or absorbed in various parts of the body enter the bloodstream and pass into the exhaled breath by gas exchange in the lungs. VOCs from skin originate from secretion of eccrine, sebaceous or apocrine glands.

It is noted, that a VOC may as well represent a derivative of a compound of interest, i.e. a compound originating from an endogenous process, or a fragment of a compound of interest, said fragment being formed by cleavage of the molecule during the analytical process.

Different methods for detecting an amount of a VOC or of a multitude of VOCs in a sample of exhaled breath or of gaseous emanation are known. According to a first embodiment, the volatile organic marker is detected via mass spectrometry, i.e. measuring the so-called mass-to-charge ratio (m/z) of the ionized compounds. In a further embodiment, the compounds are detected using proton transfer reaction time of flight mass spectrometry (PTR-ToF-MS) which comprises chemical ionization of the sample's compounds based on proton transfer. Typically, H₃O⁺ ions are used for protonation of VOCs with adequate proton affinity. The PTR-ToF-MS enables a direct analysis without sample preparation and with a high sensitivity as well as a fast response.

Alternative detection methods to detect a volatile organic marker in real-time are Selected Ion Flow Tube Mass Spectrometry (SIFT-MS) or secondary electrospray ionization high-resolution mass spectrometry (SESI-HRMS).

The sample of exhaled breath is captured using a mouthpiece, a nasal cannula, a handheld breath analyzer or any other device suitable to capture at least a fraction of the exhaled breath.

To detect an amount of one or more volatile organic marker from other gaseous emanation a sample of the secretion of one or more parts of the body is captured, e.g. from the head, chest, back, armpit, waist, arm or genital area.

It is understood that the detected amount of each volatile organic marker comprises a single value or a multitude of values over time wherein each value represents a single measurement or an average over two or more measurements. It is also understood that the amount of the volatile organic marker is either determined in absolute values or in relative values, e.g. determining an amount of change of the marker concentration.

The person's blood glucose level may be determined from the detected amount of one or more volatile organic markers in the exhaled breath or other emanation due to the correlation between said entities. It is understood that the correlation provides for values of blood glucose derived from absolute or relative values of the volatile organic marker concentration in the person's breath.

According to an embodiment, the marker data is normalized based on a reference signal, e.g. derived from ambient air or from another marker in the same sample of exhaled breath or from another sample of the person's breath or other emanation. In a further embodiment the set-up to perform the method is taught and/or trained based on individual metabolic processes, e.g. creating individual profiles for a person/patient. For example, the training involves selecting the volatile organic marker from a group of volatile organic markers also comprising derivatives or fragments of endogenous compounds.

Surprisingly, a negative correlation between the volatile organic marker amount and the blood glucose level has proven to be particularly beneficial for the reliability, responsiveness and sensitivity of the method.

The negative correlation may only be achieved by compounds that do have a functional role in the insulin response. The amount of such compounds, i.e. markers, in breath or other emanation are essentially independent of external influences or individual characteristics of the patient.

The volatile organic marker is one of indole (C₈H₇N), a partly saturated derivative of indole, a fully saturated derivative of indole and a true fraction of indole.

Indole is an aromatic heterocyclic organic compound with formula C₈H₇N. It is widely distributed in the natural environment and can be produced by a variety of bacteria, such as E. coli, which are usually present in the human intestine. Furthermore, indole is the most abundant metabolite produced from digestion of tryptophan. As many bacteria present in the human intestine can synthesize indole from tryptophan due to the enzyme tryptophanase the indole level in the human intestine is constant.

Moreover, indole is a possible signaling molecule for stimulation of Glucagon-Like-Protein-1 (GLP-1), which is needed for an accurate insulin response. In the presence of glucose, indole diffuses into intestinal cells and increases GLP-1 secretion by blocking K⁺ channels. This results in a decrease of indole concentration in the extracellular space, a decrease that is then observed in breath. As soon as extracellular glucose levels drop, indole diffuses out of cells again and detaches from K⁺ channels. These reversible mechanisms indicate that indole acts as a signal molecule only and is not metabolized. A result is the inverse progression of indole compared to blood glucose levels, i.e. the negative correlation. Furthermore, said correlation enables a monitoring of the blood glucose levels in real-time as any change in the blood glucose concentration occurs simultaneously to a change of the amount of Indole. "Simultaneously" here means that the times at which a change in the two concentrations occurs are separated by a maximum of five minutes.

A derivative is a compound that is derived from a similar compound by a chemical reaction. Some derivatives of indole, e.g. aliphatic C8-amines like cyclohexyl-ethylamine (C₈H₁₉N) or octylamine (C₈H₁₇N) or isomers thereof show a corresponding correlation to blood glucose as well as the described advantage. This also holds for some fragments, e.g. benzenes, wherein fragments denominate products of fragmentation, i.e. the dissociation of energetically unstable molecular ions formed from passing the molecules in the ionization chamber of a mass spectrometer. The term "true fragment" is used to indicate that said fragment originates from the dissociation of the claimed compound, i.e. indole.

Indole has a molecular weight of ~ 117.1 g/mol. Thus, when detecting Indole via PTR-ToF-MS the mass-to-charge ratio after proton transfer for PTR-ToF-MS is m/z = 118.1 due to the additional H+.

Derivatives are for example cyclohexyl-ethylamine C₈H₁₇N with a mass-to-charge ration of m/z = 128.14 after protonation with H⁺ or octylamine C₈H₁₉N with a mass-to-charge reaction of m/z = 130.15 after protonation.

Fragments of indole are for example benzene C₆H₆ with a mass-to-charge ration of m/z = 79.055 after protonation with H⁺ and C₇H₈ with a mass-to-charge ration of m/z = 93.069 after protonation.

According to another embodiment, the method further comprises to detect at least one additional volatile organic marker non-invasively in the exhaled breath or the other gaseous emanation from the subject as additional marker data and to determine the subject's blood glucose level based on the marker data and the additional marker data, wherein the amount of the additional volatile organic marker and the blood glucose level are a positively correlated.

Pursuant to alternative embodiments, the additional volatile organic marker is one or more of carbon dioxide, nitric oxide, formaldehyde, propanol, propanoic acid, acetone, acetic acid, butanol, butyric acid, phenol and caprolactam. Alternatively, the additional volatile organic marker is a fragment or a derivative of one or more of carbon dioxide, nitric oxide, formaldehyde, propanol, propanoic acid, acetone, acetic acid, butanol, butyric acid, phenol and caprolactam. Although the additional marker data may not be as accurate and reliable they do provide additional information. In an embodiment, additional marker data of a specific additional volatile organic marker is used to indicate hypo- or hyperglycemia additionally to the determination of the blood glucose level.

According to a further embodiment the method further comprises to detect non-invasively an amount of at least one volatile organic marker in the subject's exhaled breath as well as in the subject's other gaseous emanation as marker data. Either it is detected the amount of the same volatile organic markers or of different volatile organic markers in the breath and the other gaseous emanation respectively.

According to another embodiment, the method comprises the following steps: continuously monitoring the subject's breathing over a period of time and non-invasively detecting the amount of the at least one volatile organic marker in the continuously monitored subject's breathing over time as the maker data. Furthermore, temporal alterations of an amount of at least one control marker is detected non-invasively in the continuously monitored subject's breathing over time as control marker data. A temporal correlation between the marker data and the control marker data is determined and at least one segment of the marker data over time that corresponds to a temporal period of exhalation is selected based on the temporal correlation. The subject's blood glucose level is determined based on the selected segment of the marker data. Pursuant to alternative embodiments the control marker is one of O₂, CO, CO₂, NO, N₂, and H₂O.

According to an alternative embodiment, the method further comprises to select at least one additional segment of the marker data over time based on the temporal correlation, wherein the additional segment corresponds to a temporal period of inhalation. A blank value is determined based on the additional segment and subtracted from the marker data before determining the subject's blood glucose level.

As breathing is a cyclic process, the control marker enables to monitor said chronological sequence and to distinguish periods of exhalation and periods of inhalation. The correlation of the control data with the marker data enables to select one or more segments of the marker data corresponding to a desired period of the breathing cycles. While the exhaled breath contains VOCs originating from endogenous processes, the gas sample derived from temporal periods correlated to inhalation provides background information and e.g. is used to derive a blank value.

In an alternative embodiment to derive a blank value the method comprises to detect an amount of at least one volatile organic marker in a gaseous reference sample non-invasively as reference data, to determine a blank value based on the reference data and to subtract the blank value from the marker data before determining the subject's blood glucose level. Pursuant to a further embodiment, the gaseous reference sample contains ambient air.

According to a further embodiment a non-invasive blood glucose level monitoring system for executing the method of any of the preceding claims comprises a volatile organic marker detecting system configured to detect an amount of the volatile organic marker in a sample as marker data, a vapor distribution member configured to bring a sample of exhaled breath or other gaseous emanation from a subject in contact with the volatile organic marker detecting system and a processing unit configured to determine the subject's blood glucose level based on the marker data.

These and other advantages, effects, features and objects of the inventive concept will become better understood from the description that follows

### BRIEF DESCRIPTION OF THE DRAWINGS

The advantages, effects, features and objects other than those set forth above will become more readily apparent when consideration is given to the detailed description below. In the description, reference is made to the accompanying drawings, which form a part hereof and in which there is shown by way of illustration, not limitation, embodiments of the inventive concept. The embodiments are depicted schematically and corresponding reference characters indicate corresponding parts throughout the several views of the drawings.

It should be understood, however, that the description of exemplary embodiments that follows is not intended to limit the inventive concept to the particular forms disclosed, but on the contrary, the intention is to cover advantages, effects, features and objects falling within the scope thereof as defined by the claims below. In particular, different embodiments may be realized in an isolated fashion as well as in any arbitrary feasible combination, as the skilled person will realize.

The following description refers to the following drawings, wherein:
- Fig. 1: a flow chart of the inventive method to determine a subject's blood glucose level non-invasively by analyzing exhaled breath or another gaseous emanation from the subject,
- Fig. 2: a schematic cross-section of a proton transfer reaction time of flight mass spectrometer,
- FIG. 3: shows a correlation between a person's blood glucose level and the amount of Indole in the person's exhaled breath after glucose intake,
- Fig. 4: a correlation between the blood glucose level and the amount of different VOCs in the exhaled breath after glucose intake,
- Fig. 5: a correlation between the blood glucose level and the amount of different VOCs in the exhaled breath after lactulose intake,
- Fig. 6: a correlation between the blood glucose level and the amount of Indole in the exhaled breath after lactulose intake.

### DESCRIPTION OF EXEMPLARY EMBODIMENTS

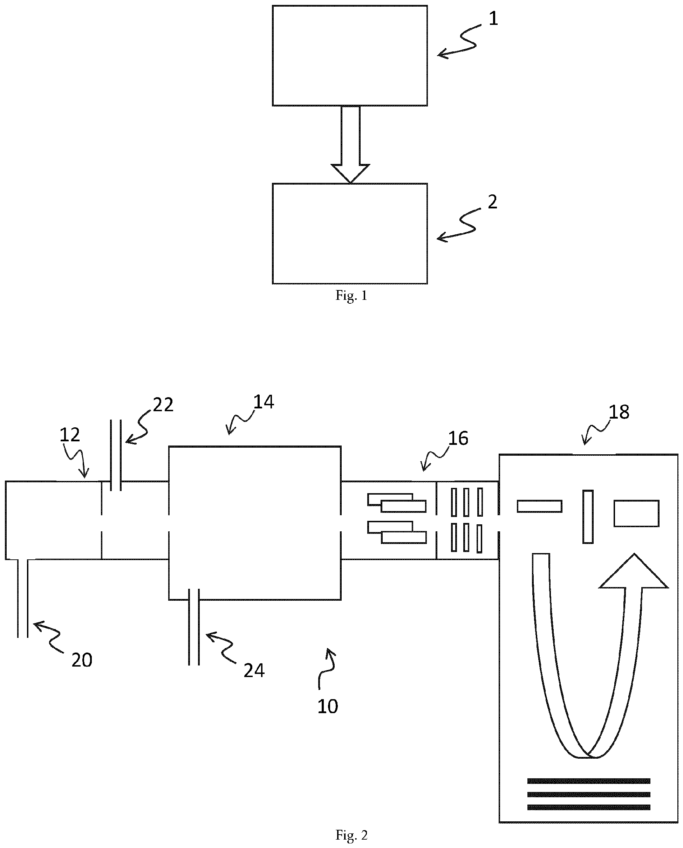

Fig. 1 depicts a flow chart of a method to determine a subject's blood glucose level non-invasively by analyzing an exhaled breath or another gaseous emanation from the subject. The method comprising at least a first step 1 of non-invasively detecting an amount of at least one volatile organic marker in the subject's exhaled breath or the subject's other gaseous emanation as marker data and a second step 2 of determining the subject's blood glucose level based on the marker data. The at least one volatile marker detected in step 1 is selected from a group of markers for which the amount of the volatile organic marker is negatively correlated to the blood glucose level. The volatile organic marker is one of indole (C8H7N), a partly saturated derivative of indole, fully saturated derivative of indole and a true fraction of indole.

Fig. 2 shows a schematic cross-section of a proton transfer reaction time of flight mass spectrometer (PTR-ToF-MS) 10 as one example of a device suitable to carry out step 1, i.e. to detect the amount of different VOCs in a sample, such as exhaled breath.

The shown mass spectrometer 10 consists of an ion source 12, a drift tube 14, a quadrupole ion guide 16 and a reflectron time of flight mass analyzer 18. Water vapor enters the ion source 12 through a water vapor inlet 20 and hydronium ions (H₃O⁺) are generated from the water vapor by a hollow cathode discharge. The ion source 12 further comprises an excess water vapor outlet 22. In the drift tube 14, the hydronium ions react in a proton transfer reaction with analytes, like the volatile organic marker to be detected, from a sample, i.e. exhaled breath of the subject that is injected through a sample gas inlet 24. In the depicted embodiment, said protonated analytes are directed towards the mass analyzer 18 through a two-part quadrupole ion guide16. In the reflectron time of flight mass analyzer 18 the ionized analytes are accelerated such that all ions have the same kinetic energy and can be separated according to their mass to charge ration m/z via a time of flight measurement.

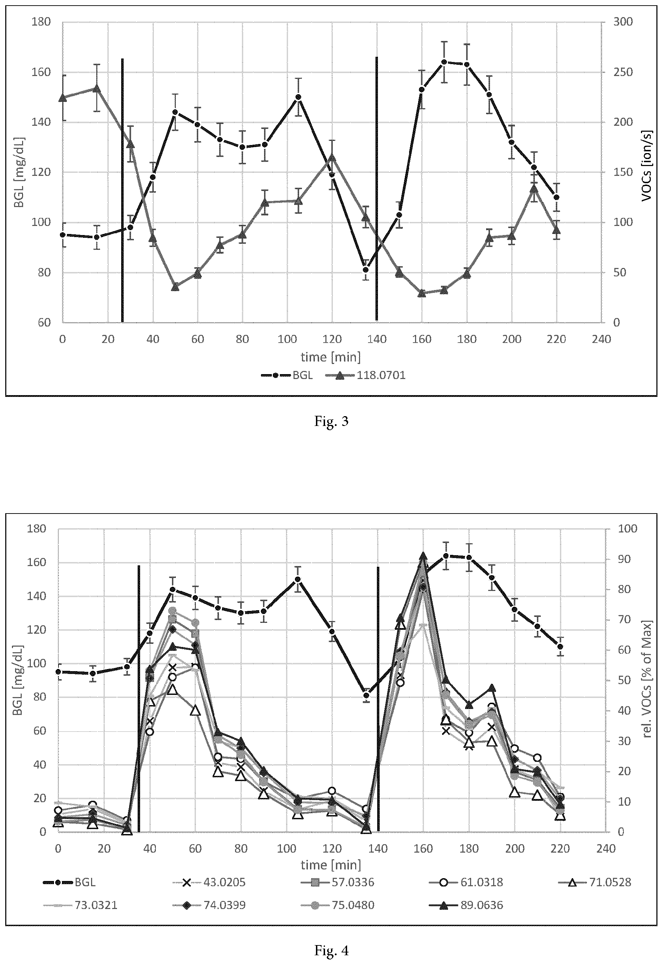

In Fig. 3 a graph is depicted showing a person's blood glucose levels BGL over time as well as the amount of Indole VOC in the person's exhaled breath over time. Over the depicted period of time the person started in a fasting state (no food intake during previous eight hours) with a blood glucose level BGL of about 90 mg/dL and ingested predefined amounts of glucose at two different points in time indicated by the vertical lines.

After each glucose intake (indicated by vertical lines), the blood glucose level BGL rises sharply to a peak value before starting to decrease again. Parallel to the increase of the blood glucose level BGL the amount of Indole VOC in the person's breath decreases and with the decrease of the blood glucose level BGL the amount of Indole VOC starts to rise again. Thus, the blood glucose level BGL and the amount of Indole VOC are negatively correlated.

In Fig. 4 a graph is depicted showing the person's blood glucose levels BGL over time as in Fig. 3 and the amount of other volatile organic markers VOCs in the person's exhaled breath over time.

Unlike Indole, the other volatile organic markers VOCs shown in this graph exhibit a positive correlation to the rise of the blood glucose level BGL. Moreover, the decrease of said other volatile organic markers occurs significantly earlier in time than the decrease of the blood glucose level.

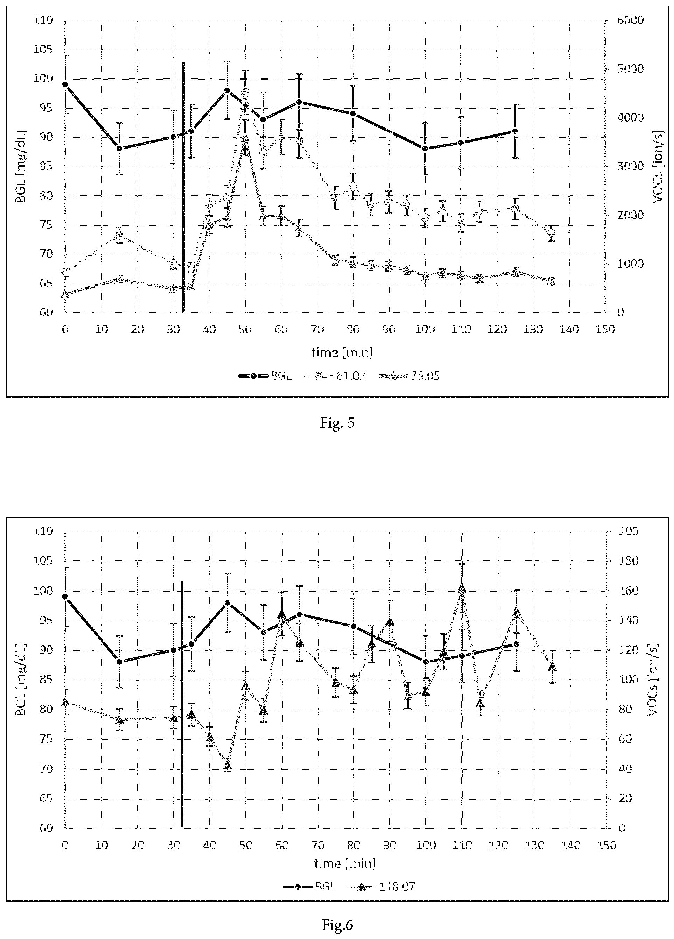

Fig. 5 and 6 depict the correlation between the blood glucose level and the amount of different volatile organic compounds in the exhaled breath after lactulose intake. Lactulose is a disaccharide consisting of D-galactose and fructose and is not metabolized, i.e. digested, by the human organism. Instead, intestinal bacteria metabolize it. Correspondingly, the blood glucose level BGL is not affected by the intake of lactulose but stays constant around 95 mg/dL. A vertical line indicates the event of the lactulose intake in the figures.

As can be seen in Fig. 5 the other volatile organic markers VOCs do show a correlation, i.e. a considerable increase correlated to the event of the lactulose intake. Thus, these volatile organic markers are at least in parts originating from gut bacteria metabolism- and not directly correlated to the blood glucose metabolism. Therefore, the amount of these other organic markers is not optimal for blood glucose monitoring as it may be affected by external influences.

However, the amount of Indole VOC in the person's breath is not affected by the lactulose intake, i.e. it remains constant like the glucose level, as can be seen in Fig. 6. Thus, indole is not linked to a gut bacteria metabolism but to the glucose metabolism, and as such a suitable biomarker for glucose monitoring, untainted with external influences.

## Claims

1. A computer-implemented method to determine a subject's blood glucose level non-invasively by analyzing an exhaled breath or another gaseous emanation from the subject, comprising:
- non-invasively detecting an amount of at least one volatile organic marker in the subject's exhaled breath or the subject's other gaseous emanation as marker data;
- determining the subject's blood glucose level based on the marker data;
- wherein the volatile organic marker is selected from a group of markers for which the amount of the volatile organic marker is negatively correlated to the blood glucose level
**characterized in that**
the volatile organic marker is one of indole (C8H7N), a partly saturated derivative of indole, fully saturated derivative of indole and a true fraction of indole.

2. The method of claim 1, wherein the amount of the volatile organic marker is detected by mass spectrometry.

3. The method of any of the preceding claims ,wherein the method further comprises:
- non-invasively detecting at least one additional volatile organic marker in the exhaled breath or the other gaseous emanation from the subject as additional marker data,
- determining the subject's blood glucose level based on the marker data and the additional marker data,
- wherein the additional volatile organic marker is selected from a group of markers for which the amount of the volatile organic marker is positively correlated to the blood glucose level.

4. The method of claim 3, wherein the additional volatile organic marker is one or more of carbon dioxide, nitric oxide, formaldehyde, propanol, propanoic acid, acetone, acetic acid, butanol, butyric acid, phenol and caprolactam.

5. The method of claim 3 or 4, wherein the additional volatile organic marker is a derivative or a fragment of one or more of carbon dioxide, nitric oxide, formaldehyde, propanol, propanoic acid, acetone, acetic acid, butanol, butyric acid, phenol and caprolactam.

6. The method of any of the preceding claims wherein the method further comprises:
- non-invasively detecting an amount of at least one volatile organic marker in the subject's exhaled breath as well as in the subject's other gaseous emanation as marker data.

7. The method of any of the preceding claims wherein the method comprises:
- continuously monitoring the subject's breathing over a period of time,
- non-invasively detecting the amount of the at least one volatile organic marker in the continuously monitored subject's breathing over time as the maker data,
- non-invasively detecting temporal alterations of an amount of at least one control marker in the continuously monitored subject's breathing over time as control marker data,
- determining a temporal correlation between the marker data and the control marker data,
- selecting at least one segment of the marker data over time corresponding to a temporal period of exhalation based on the temporal correlation and
- determining the subject's blood glucose level based on the selected segment of the marker data.

8. The method of claim 7 wherein the control marker is one of O₂, CO, CO₂, NO, N₂, and H₂O.

9. The method of claim 7 or 8 wherein the method comprises:
- selecting at least one additional segment of the marker data over time corresponding to a temporal period of inhalation based on the temporal correlation,
- determining a blank value based on the additional segment and
- subtracting the blank value from the marker data before determining the subject's blood glucose level.

10. The method of any of the preceding claims wherein the method comprises:
- non-invasively detecting an amount of at least one volatile organic marker in a gaseous reference sample as reference data,
- determining a blank value based on the reference data and
- subtracting the blank value from the marker data before determining the subject's blood glucose level.

11. A non-invasive blood glucose level monitoring system for executing the method of any of the preceding claims comprising:
- a volatile organic marker detecting system configured to detect an amount of the volatile organic marker in a sample as marker data,
- a vapor distribution member configured to bring a sample of exhaled breath or other gaseous emanation from a subject in contact with the volatile organic marker detecting system and
- a processing unit configured to determine the subject's blood glucose level based on the marker data.

## Patentansprüche

1. Computerimplementiertes Verfahren zum nicht invasiven Bestimmen eines Blutglukosespiegels eines Individuums durch Analysieren einer Ausatemluft oder einer anderen gasförmigen Emanation aus dem Individuum, umfassend:
- nicht invasives Nachweisen einer Menge von mindestens einem flüchtigen organischen Marker in der Ausatemluft des Individuums oder der anderen gasförmigen Emanation des Individuums als Markerdaten;
- Bestimmen des Blutglukosespiegels des Individuums basierend auf den Markerdaten;
- wobei der flüchtige organische Marker aus einer Gruppe von Markern ausgewählt ist, für die die Menge des flüchtigen organischen Markers negativ mit dem Blutglukosespiegel korreliert;
**dadurch gekennzeichnet, dass**
der flüchtige organische Marker einer von Indol (C8H7N), einem teilweise gesättigten Derivat von Indol, einem vollständig gesättigten Derivat von Indol und einer wahren Fraktion von Indol ist.

2. Verfahren nach Anspruch 1, wobei die Menge des flüchtigen organischen Markers mittels Massenspektrometrie nachgewiesen wird.

3. Verfahren nach einem der vorstehenden Ansprüche, wobei das Verfahren ferner Folgendes umfasst:
- nicht invasives Nachweisen von mindestens einem zusätzlichen flüchtigen organischen Marker in der Ausatemluft oder der anderen gasförmigen Emanation aus dem Individuum als zusätzliche Markerdaten,
- Bestimmen des Blutglukosespiegels des Individuums basierend auf den Markerdaten und den zusätzlichen Markerdaten,
- wobei der zusätzliche flüchtige organische Marker aus einer Gruppe von Markern ausgewählt ist, für die die Menge des flüchtigen organischen Markers positiv mit dem Blutglukosespiegel korreliert.

4. Verfahren nach Anspruch 3, wobei der zusätzliche flüchtige organische Marker einer oder mehrere von Kohlendioxid, Stickoxid, Formaldehyd, Propanol, Propansäure, Aceton, Essigsäure, Butanol, Buttersäure, Phenol und Caprolactam ist.

5. Verfahren nach Anspruch 3 oder 4, wobei der zusätzliche flüchtige organische Marker ein Derivat oder ein Fragment von einem oder mehreren von Kohlendioxid, Stickoxid, Formaldehyd, Propanol, Propansäure, Aceton, Essigsäure, Butanol, Buttersäure, Phenol und Caprolactam ist.

6. Verfahren nach einem der vorstehenden Ansprüche, wobei das Verfahren ferner Folgendes umfasst:
- nicht invasives Nachweisen einer Menge von mindestens einem flüchtigen organischen Marker in der Ausatemluft des Individuums sowie in der anderen gasförmigen Emanation des Individuums als Markerdaten.

7. Verfahren nach einem der vorstehenden Ansprüche, wobei das Verfahren Folgendes umfasst:
- kontinuierliches Überwachen der Atmung des Individuums über einen Zeitraum,
- nicht invasives Nachweisen der Menge des mindestens einen flüchtigen organischen Markers in der kontinuierlich überwachten Atmung des Individuums im Laufe der Zeit als die Markerdaten,
- nicht invasives Nachweisen temporaler Veränderungen einer Menge von mindestens einem Kontrollmarker in der kontinuierlich überwachten Atmung des Individuums im Laufe der Zeit als Kontrollmarkerdaten,
- Bestimmen einer temporalen Korrelation zwischen den Markerdaten und den Kontrollmarkerdaten,
- Auswählen mindestens eines Segments der Markerdaten im Laufe der Zeit, das einer temporalen Ausatmungsdauer entspricht, basierend auf der temporalen Korrelation; und
- Bestimmen des Blutglukosespiegels des Individuums basierend auf dem ausgewählten Segment der Markerdaten.

8. Verfahren nach Anspruch 7, wobei der Kontrollmarker einer von O₂, CO, CO₂, NO, N₂, und H₂O ist.

9. Verfahren nach Anspruch 7 oder 8, wobei das Verfahren Folgendes umfasst:
- Auswählen mindestens eines zusätzlichen Segments der Markerdaten im Laufe der Zeit, das einer temporalen Einatmungsdauer entspricht, basierend auf der temporalen Korrelation,
- Bestimmen eines Blindwertes basierend auf dem zusätzlichen Segment; und
- Subtrahieren des Blindwertes von den Markerdaten vor dem Bestimmen des Blutglukosespiegels des Individuums.

10. Verfahren nach einem der vorstehenden Ansprüche, wobei das Verfahren Folgendes umfasst:
- nicht invasives Nachweisen einer Menge von mindestens einem flüchtigen organischen Marker in einer gasförmigen Referenzprobe als Referenzdaten,
- Bestimmen eines Blindwertes basierend auf den Referenzdaten; und
- Subtrahieren des Blindwertes von den Markerdaten vor dem Bestimmen des Blutglukosespiegels des Individuums.

11. Nicht invasives Blutglukosespiegelüberwachungssystem zum Ausführen des Verfahrens nach einem der vorstehenden Ansprüche, umfassend:
- ein Nachweissystem für einen flüchtigen organischen Marker, das zum Nachweisen einer Menge des flüchtigen organischen Markers in einer Probe als Markerdaten ausgelegt ist,
- ein Dampfverteilungselement, das zum Inkontaktbringen einer Probe von Ausatemluft oder einer anderen gasförmigen Emanation von einem Individuum mit dem Nachweissystem für einen flüchtigen organischen Marker ausgelegt ist; und
- eine Verarbeitungseinheit, die zum Bestimmen des Blutglukosespiegels des Individuums basierend auf den Markerdaten ausgelegt ist.

## Revendications

1. Procédé mis en oeuvre par ordinateur pour déterminer le niveau de glucose sanguin d'un sujet de manière non invasive en analysant un air expiré ou une autre émanation gazeuse provenant du sujet, comprenant :
- la détection de manière non invasive d'une quantité d'au moins un marqueur organique volatil dans l'air expiré du sujet ou l'autre émanation gazeuse du sujet en tant que données du marqueur ;
- la détermination du niveau de glucose sanguin du sujet sur la base des données du marqueur ;
- dans lequel le marqueur organique volatil est sélectionné dans un groupe de marqueurs pour lequel la quantité du marqueur organique volatil est corrélée de manière négative au niveau de glucose sanguin
**caractérisé en ce que**
le marqueur organique volatil est un parmi l'indole (C8H7N), un dérivé partiellement saturé de l'indole, un dérivé entièrement saturé de l'indole et une fraction authentique de l'indole.

2. Procédé selon la revendication 1, dans lequel la quantité du marqueur organique volatil est détectée par spectrométrie de masse.

3. Procédé selon l'une quelconque des revendications précédentes, dans lequel le procédé comprend en outre :
- la détection de manière non invasive d'au moins un marqueur organique volatil supplémentaire dans l'air expiré ou l'autre émanation gazeuse provenant du sujet en tant que données du marqueur supplémentaire,
- la détermination du niveau de glucose sanguin du sujet sur la base des données du marqueur et des données du marqueur supplémentaire,
- dans lequel le marqueur organique volatil supplémentaire est sélectionné dans un groupe de marqueurs pour lequel la quantité du marqueur organique volatil est corrélée de manière positive au niveau de glucose sanguin.

4. Procédé selon la revendication 3, dans lequel le marqueur organique volatil supplémentaire est un ou plusieurs parmi le dioxyde de carbone, l'oxyde nitrique, le formaldéhyde, le propanol, l'acide propanoïque, l'acétone, l'acide acétique, le butanol, l'acide butyrique, le phénol et le caprolactame.

5. Procédé selon la revendication 3 ou 4, dans lequel le marqueur organique volatil supplémentaire est un dérivé ou un fragment d'un ou de plusieurs parmi le dioxyde de carbone, l'oxyde nitrique, le formaldéhyde, le propanol, l'acide propanoïque, l'acétone, l'acide acétique, le butanol, l'acide butyrique, le phénol et le caprolactame.

6. Procédé selon l'une quelconque des revendications précédentes dans lequel le procédé comprend en outre :
- la détection de manière non invasive d'une quantité d'au moins un marqueur organique volatil dans l'air expiré du sujet ainsi que dans l'autre émanation gazeuse du sujet en tant que données du marqueur.

7. Procédé selon l'une quelconque des revendications précédentes dans lequel le procédé comprend :
- la surveillance de manière continue de la respiration du sujet sur une période de temps,
- la détection de manière non invasive de la quantité de l'au moins un marqueur organique volatil dans la respiration du sujet surveillée de manière continue au cours du temps en tant que données du marqueur,
- la détection de manière non invasive d'altérations temporelles d'une quantité d'au moins un marqueur de contrôle dans la respiration du sujet surveillée de manière continue au cours du temps en tant que données du marqueur de contrôle,
- la détermination d'une corrélation temporelle entre les données du marqueur et les données du marqueur de contrôle,
- la sélection d'au moins un segment des données du marqueur au cours du temps correspondant à une période temporelle d'expiration sur la base de la corrélation temporelle et
- la détermination du niveau de glucose sanguin du sujet sur la base du segment sélectionné des données du marqueur.

8. Procédé selon la revendication 7 dans lequel le marqueur de contrôle est un parmi O₂, CO, CO₂, NO, N₂ et H₂O.

9. Procédé selon la revendication 7 ou 8 dans lequel le procédé comprend :
- la sélection d'au moins un segment supplémentaire des données du marqueur au cours du temps correspondant à une période temporelle d'inspiration sur la base de la corrélation temporelle,
- la détermination d'une valeur à blanc sur la base du segment supplémentaire et
- la soustraction de la valeur à blanc des données du marqueur avant la détermination du niveau de glucose sanguin du sujet.

10. Procédé selon l'une quelconque des revendications précédentes dans lequel le procédé comprend :
- la détection de manière non invasive d'une quantité d'au moins un marqueur organique volatil dans un échantillon de référence gazeux en tant que données de référence,
- la détermination d'une valeur à blanc sur la base des données de référence et
- la soustraction de la valeur à blanc des données du marqueur avant la détermination du niveau de glucose sanguin du sujet.

11. Système de surveillance de niveau de glucose sanguin non invasif destiné à l'exécution du procédé selon l'une quelconque des revendications précédentes comprenant :
- un système de détection de marqueur organique volatil configuré pour détecter une quantité du marqueur organique volatil dans un échantillon en tant que données du marqueur,
- un élément de distribution de vapeur configuré pour amener un échantillon d'air expiré ou d'une autre émanation gazeuse provenant d'un sujet en contact avec le système de détection de marqueur organique volatil et
- une unité de traitement configurée pour déterminer le niveau de glucose sanguin du sujet sur la base des données du marqueur.
